# EUROPEAN PATENT SPECIFICATION

(11) **EP 2 877 093 B1**
(45) Date of publication and mention of the grant of the patent: **30.12.2020**
(21) Application number: 13745027.6
(22) Date of filing: 25.07.2013
(51) Int. Cl.: A61B 5/145

(54) **HANDHELD MEDICAL DEVICE FUNCTIONALITY WITHOUT BATTERY**
BATTERIELOSE FUNKTION EINER TRAGBAREN MEDIZINISCHEN VORRICHTUNG
FONCTIONNALITÉ DE DISPOSITIF MÉDICAL MANUEL SANS BATTERIE

(30) Priority: 27.07.2012 US 201213559648
(43) Date of publication of application: 03.06.2015
(73) Proprietor: Roche Diabetes Care GmbH, 68305 Mannheim (DE); F. Hoffmann-La Roche AG, 4070 Basel (CH)
(72) Inventor: MCKEE, Michael C., Arlington Heights, Illinois 60004 (US); NICHOLAS, Michael G., Wheeling, Illinois 60090-2453 (US); RAMEY, Blaine E., Indianapolis, Indiana 46204 (US)
(74) Representative: Freiherr von Campenhausen, Harald
(86) International application number: PCT/EP2013/065763
(87) International publication number: WO 2014/016392

(56) References cited:
- WO-A2-2010/040090
- GB-A- 2 474 261
- US-A1- 2007 040 449
- US-A1- 2011 009 824
- Mark Cantrell: "Simplify USB Isolation in Medical Applications", , 31 March 2010 (2010-03-31), XP055254927, Retrieved from the Internet: URL:http://www.ecnmag.com/article/2010/03/ simplify-usb-isolation-medical-application s [retrieved on 2016-03-03]

## Description

### FIELD

The present disclosure relates to handheld medical devices and more particularly to functionality of handheld medical devices with and without batteries.

### BACKGROUND

Diabetes mellitus, often referred to as diabetes, is a chronic condition in which a person has elevated blood glucose levels that result from defects in the body's ability to produce and/or use insulin. There are three main types of diabetes. Type 1 diabetes usually strikes children and young adults, and can be autoimmune, genetic, and/or environmental. Type 2 diabetes accounts for 90-95% of diabetes cases and is linked to obesity and physical inactivity. Gestational diabetes is a form of glucose intolerance diagnosed during pregnancy and usually resolves spontaneously after delivery.

In 2009, according to the World Health Organization, at least 220 million people worldwide suffer from diabetes. In 2005, an estimated 1.1 million people died from diabetes. The incidence of diabetes is increasing rapidly, and it is estimated that between 2005 and 2030, the number of deaths from diabetes will double. In the United States, nearly 24 million Americans have diabetes with an estimated 25 percent of seniors age 60 and older being affected. The Centers for Disease Control and Prevention forecast that 1 in 3 Americans born after 2000 will develop diabetes during their lifetime. The National Diabetes Information Clearinghouse estimates that diabetes costs $132 billion in the United States alone every year. Without treatment, diabetes can lead to severe complications such as heart disease, stroke, blindness, kidney failure, amputations, and death related to pneumonia and flu.

Management of diabetes is complex because the level of blood glucose entering the bloodstream is dynamic. Variation of insulin in the bloodstream that controls the transport of glucose out of the bloodstream also complicates diabetes management. Blood glucose levels are sensitive to diet and exercise, but also can be affected by sleep, stress, smoking, travel, illness, menses, and other psychological and lifestyle factors that are unique to each patient. The dynamic nature of blood glucose and insulin, and all other factors affecting blood glucose, often require a person with diabetes to forecast blood glucose levels. Administration of insulin and/or oral medications can be regulated and timed to maintain blood glucose levels within an appropriate range at all times.

Management of diabetes is often highly intrusive because of the need to consistently obtain reliable diagnostic information, follow prescribed therapy, and manage lifestyle on a daily basis. Diagnostic information, such blood glucose level, can be obtained from a capillary blood sample with a lancing device and a test strip. The blood glucose level is measured via the test strip using a handheld blood glucose meter. Interstitial glucose levels can be obtained from a continuous glucose sensor worn on the body.

A therapy regimen for a patient can be established based on one or more of the patient's blood glucose levels. The therapy regimen can include administration of insulin and/or oral medication. Insulin can be administered with a syringe, an insulin pen, an ambulatory infusion pump, or a combination of two or more of the above. With insulin therapy, determining the amount of insulin to inject at a given time can require forecasting meal amount and composition (e.g., of fat, carbohydrates, and proteins, and amounts of each). Determining the amount of insulin to inject at a given time can also require consideration of the effects of exercise and physiologic state. The patient's management of lifestyle factors such as body weight, diet, and exercise can significantly influence the type and effectiveness of therapy.

Management of diabetes involves large amounts of diagnostic data and prescriptive data that are acquired from medical devices, personal health care devices, patient recorded information, health care professional tests results, prescribed medications and recorded information. Medical devices including self-monitoring bG meters, continuous glucose monitors, insulin infusion pumps, diabetes analysis software, and diabetes device configuration software each of which generates or manages or both large amounts of diagnostic and prescriptive data. Personal health care devices can include weights, scales, blood pressure cuffs, pedometers, other activity monitors, and other suitable devices. Patient recorded data can include information relating to meals, exercise, and lifestyle. Health care professional biomarker data can include HbA1C, cholesterol, triglycerides, fasting glucose, and glucose tolerance. Health care professional recorded information can include therapy and other patient-specific information.

Handheld diabetes management devices include one or more batteries that power the handheld diabetes management device. For example, some handheld diabetes management devices include replaceable, standard size batteries and some handheld diabetes management devices include a non-standard sized, re-chargeable battery. Handheld diabetes management devices that include a re-chargeable battery generally include provisions for a connection to one or more external power sources for re-charging the battery.

If the battery is disconnected from a handheld diabetes management device, the handheld diabetes management device generally is inoperable, even when connected to an external power source. However, a user of the handheld diabetes management device may desire to operate the handheld diabetes management device when the battery is disconnected from the handheld diabetes management device, such as when the battery has been lost. Additionally, it may be necessary to operate the device when the battery is disconnected from the handheld diabetes management device, such as for testing during manufacturing of the handheld diabetes management device. Thus, there is a need for a handheld diabetes management device that is at least partially operable without its battery.

GB 2474261 A describes an apparatus for regulating charge from a connected power supply to a chargeable power source such as a battery and one or more components of an electronic device. The apparatus is configured to have one or more powering modes. WO 2010/040090 A2 describes a fluid analyte meter which can also include a battery that may be recharged by a connection via a USB interface element to either a processing device, such as a PC, or other external power supply. US 2007/040449 A1 describes a powering subassembly for a portable electronic device includes a main battery, a converter, a backup battery, and a controller. The main battery provides a main voltage and the backup battery can provide a backup voltage. US 2011/009824 A1 discloses a portable ambulatory fluid delivery device including a dispensing unit including one or more rechargeable batteries, a housing to retain the one or more rechargeable batteries, and at least one electrical connector to be coupled to a recharging unit to direct electrical power received from the recharging unit to recharge the one or more rechargeable batteries. The publication by Cantrell, M., entitled "Simplify USB Isolation in Medical Applications", 31 March 2010, is also relevant for understanding the background of the invention.

The background description provided herein is for the purpose of generally presenting the context of the disclosure. Work of the presently named inventors, to the extent it is described in this background section, as well as aspects of the description that cannot otherwise qualify as prior art at the time of filing, are neither expressly nor impliedly admitted as prior art against the present disclosure.

SUMMARYThe present invention is as defined in the claims.

It should be understood that the detailed description and specific examples are intended for purposes of illustration only and are not intended to limit the scope of the disclosure.

### BRIEF DESCRIPTION OF THE DRAWINGS

The present disclosure will become more fully understood from the detailed description and the accompanying drawings, wherein:
FIG. 1 shows a patient and a health care professional along with various devices that can be used to help the patient monitor and control health;
FIG. 2 shows a patient with a continuous glucose monitor (CGM), an ambulatory durable insulin infusion pump, an ambulatory non-durable insulin infusion pump, and a blood glucose (bG) management device;
FIG. 3 shows a diabetes care system of systems that can be used to manage diabetes;
FIG. 4 is a front view of an example implementation of a handheld diabetes management device;
FIG. 5 is a side view of the example handheld diabetes management device;
FIG. 6 is a functional block diagram of a portion of the example handheld diabetes management device; and
FIGs. 7A and 7B are illustrations of the functional block diagram of FIG. 6 with current paths through the example handheld diabetes management device.

### DETAILED DESCRIPTION

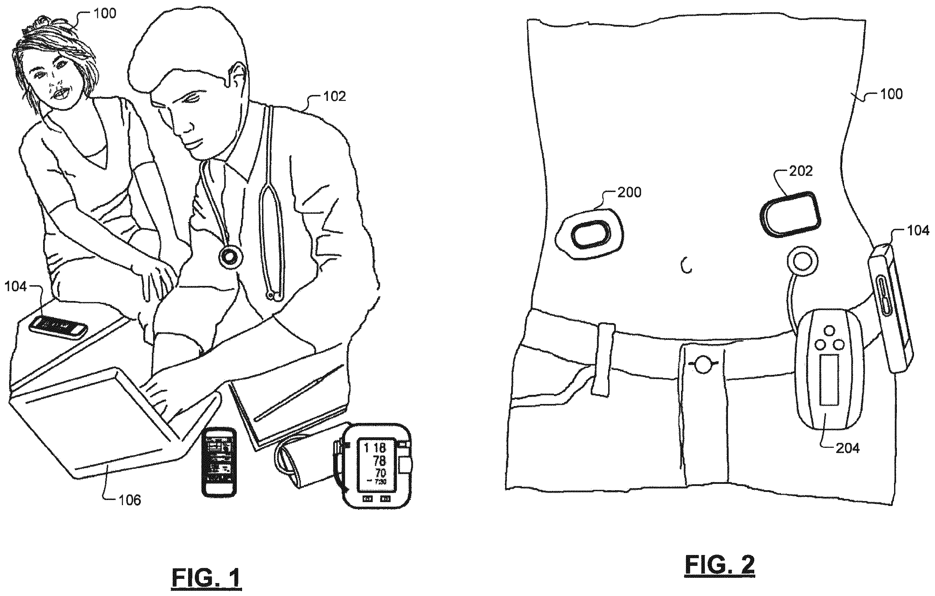

Referring now to FIG. 1, a patient 100 with diabetes and a health care professional 102 are shown in a clinical environment. The patient 100 with diabetes can be diagnosed with a metabolic syndrome, pre-diabetes, type 1 diabetes, type 2 diabetes, gestational diabetes, etc. Healthcare providers for diabetes are diverse and include nurses, nurse practitioners, physicians, endocrinologists, and others and are collectively referred to as health care professionals.

During a health care consultation, the patient 100 typically shares with the health care professional 102 a variety of data including blood glucose (bG) measurements, continuous glucose monitor data, amounts and type of insulin administered, amounts of food and beverages consumed, exercise schedules, health status, and other lifestyle information. The health care professional 102 can obtain additional data for the patient 100, such as measurements of HbA1C, cholesterol levels, plasma glucose, triglycerides, blood pressure, and weight. The data can be recorded manually or electronically on a handheld diabetes management device 104 (e.g., a handheld bG monitor device), a diabetes analysis software executed on a personal computer (PC) 106, and/or a web-based diabetes analysis site. The health care professional 102 can analyze the patient data manually or electronically using the diabetes analysis software and/or the web-based diabetes analysis site. After analyzing the data and reviewing how efficacious previously prescribed therapy is and how well the patient 100 followed the previously prescribed therapy, the health care professional 102 can decide whether to modify a therapy prescribed for the patient 100.

Referring now to FIG. 2, the patient 100 can use a continuous glucose monitor (CGM) 200, an ambulatory durable insulin infusion pump 204 or an ambulatory non-durable insulin infusion pump 202 (collectively insulin pump 204), and the diabetes management device 104. The CGM 200 can use a subcutaneous sensor to sense and monitor the amount of glucose (e.g., glucose concentration) of the patient 100. The CGM 200 communicates glucose measurements to the diabetes management device 104.

The diabetes management device 104 performs various tasks including measuring and recording bG measurements, determining an amount of insulin to be administered to the patient 100 via the insulin pump 204, receiving user input via a user interface, archiving data, performing structured bG tests, etc. The diabetes management device 104 can transmit instructions to the insulin pump 204, and the insulin pump 204 selectively delivers insulin to the patient 100. Insulin can be delivered in the form of a meal bolus dose, a correction bolus dose, a basal dose, etc.

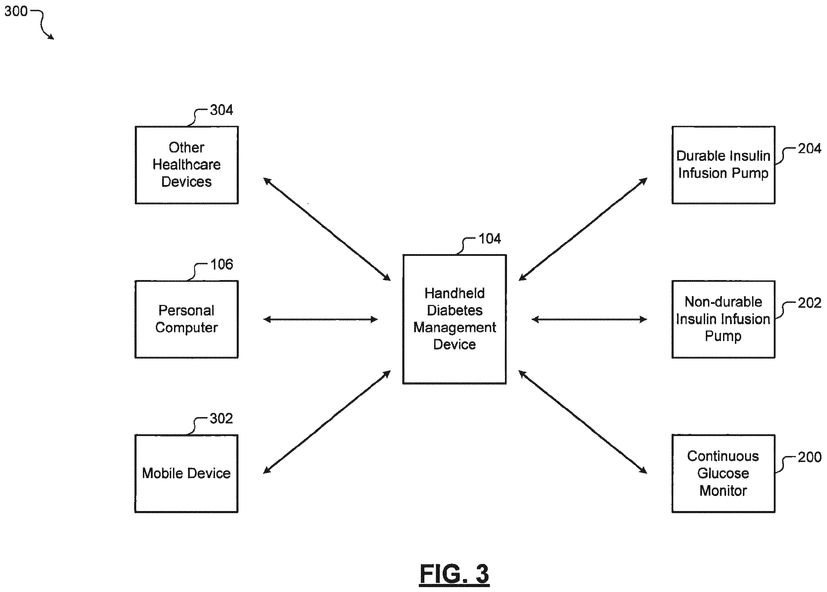

Referring now to FIG. 3, a diabetes management system 300 is shown which can be used by the patient 100 and/or the health care professional 102. The system 300 can include one or more of the following devices: the diabetes management device 104, the CGM 200, the insulin pump 204, a mobile device 302, the diabetes management software executed on the computer 106, and one or more other health care devices 304. The diabetes management device 104 can be configured as a system "hub" and communicate with one or more of the other devices of the system 300. The insulin pump 204, the mobile device 302, or another suitable device can alternatively serve as the system hub. Communication between various devices in the system 300 can be performed using wireless interfaces (e.g., Bluetooth) and/or wired interfaces (e.g., USB). Communication protocols used by these devices can include protocols compliant with the IEEE 11073 standard as extended using guidelines provided by Continua Health Alliance Design Guidelines. Further, health care records systems such as Microsoft HealthVault™ and Google Health™ can be used by the patient 100 and the health care professional 102 to exchange information.

The diabetes management software running on the computer 106 can include an analyzer-configurator that stores configuration information for devices of the system 300. For example only, the configurator has a database to store configuration information for the diabetes management device 104 and the other devices. A patient can interface the configurator through standard web based or computer graphical user interfaces (GUIs). The configurator selectively transmits patient-approved configurations to the devices of the system 300. The analyzer selectively retrieves data from the devices of the system 300, stores the data in a database, selectively analyzes the data, and outputs analysis results through standard web based or computer GUIs.

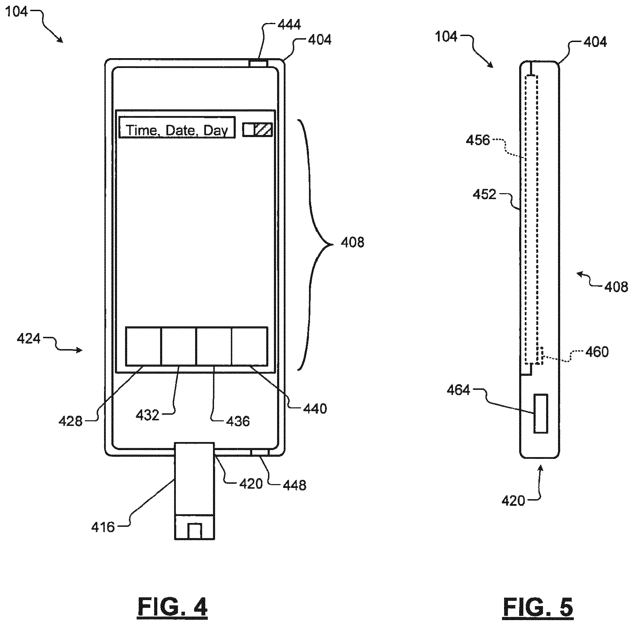

Referring now to FIG. 4, a high level illustration of an example embodiment of the diabetes management device 104 is presented. The diabetes management device 104 includes, among other things, a housing 404, user unit control switches (not specifically numbered), a touchscreen display 408, and a bG test strip port 420. The user unit control switches, for example, can include ON/OFF switches, volume switches, alarm switches for bG testing and/or insulin administration, and/or one or more other switches or other types of control devices that a patient can use to control functions/operations of the diabetes management device 104.

A bG test strip 416 can be inserted into the bG test strip port 420. The bG test strip 416 can be inserted into the bG test strip port 420 by a patient, from a test strip drum (not shown) located within the housing 404, or in another suitable manner. The bG test strip 416 is shown already inserted into the bG test strip port 420 in the example of FIG. 4.

User selectable options 424 can be displayed on a portion of the display 408. The selectable options 424 can include a menu option 428, a bolus insulin option 432, a carbohydrate option 436, and an event option 440. One or more other user selectable options can additionally or alternatively be available. The patient can access a device menu for the diabetes management device 104 by selecting the menu option 428. The patient can input various insulin (and/or other medication) information (e.g., amount, insulin type, etc.) by selecting the bolus insulin option 432. The patient can input various carbohydrate intake information (e.g., amount) by selecting the carbohydrate option 436. The patient can also input other food intake information (e.g., protein content, fat content, etc.) by selecting the carbohydrate option 436. The patient can input various event related information (e.g., meals, exercise, periods of stress, etc.) that can affect the patient's bG measurements by selecting the event option 440. The patient may access and view a history of events (e.g., of bG measurements, exercise, carbohydrate intake, insulin administration, etc.) via the menu option 428.

Although the display 408 is described herein as a touchscreen display, the diabetes management device 104 can include another suitable form of display (e.g., LED, etc.). If a touchscreen display is not used, the user control switches can include specific buttons or controls by which the patient is able to select various options and input markers needed to operate the diabetes management device 104. The diabetes management device 104 may include one or more visual indicators, such as LED 444. The diabetes management device 104 may include one or more audio output devices, such as speaker 448.

FIG. 5 includes a side view of the example diabetes management device 104. Referring now to FIGs. 4 and 5, the diabetes management device 104 may include a battery compartment 452 for housing a re-chargeable battery 456. The battery 456 may include, for example, a lithium based (e.g., lithium ion) battery or another suitable type of, in particular re-chargeable, battery. The diabetes management device 104 includes battery terminals 460 for connecting the battery 456 with the diabetes management device 104. The diabetes management device 104 can operate using power drawn from the battery 456.

The diabetes management device 104 can also operate using power drawn from a power source that is external to the diabetes management device 104. The diabetes management device 104 includes a port 464 that is configured in accordance with a universal serial bus (USB) standard. The port 464 is configured to receive a plug (not shown) that is also configured in accordance with the USB standard. The port 464 may be configured to receive, for example, a t standard, mini, or micro type USB plug that is configured in accordance with, for example, USB 1.x, 2.x, or 3.x standards or another suitable protocol.

When the battery 456 is connected with the battery terminals 460, the battery 456 can be re-charged via power received via the port 464. More specifically, the plug is part of a cable that, at its other end, can be plugged into, for example, a USB port of an adapter that can be plugged into a power source (e.g., a standard wall outlet, a vehicle, etc.) or a USB port of a computer, to receive power. The diabetes management device 104 can operate using power drawn from the battery 456 and/or the port 464 when the battery 456 is connected to the battery terminals 460. When the battery 456 is not connected to the battery terminals 460, the diabetes management device 104 can, at least partially, operate using power drawn via the port 464.

The above description is a broad description of the diabetes management device 104. In practice, the diabetes management device 104 can include additional controls, input ports, output ports, etc., as can be desired to further enhance its utility or its use with other components and devices (e.g., computers, infusion pumps, cellular phones, etc.). The description of the diabetes management device 104 should not be taken as limiting as to the construction of the diabetes management device 104 or as to the features and capabilities of the diabetes management device 104.

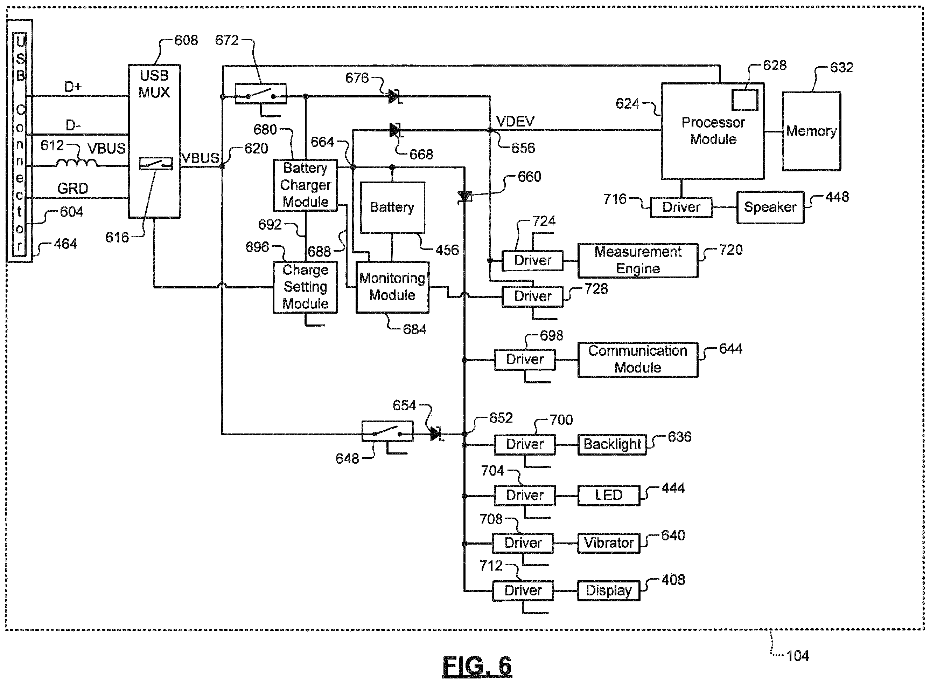

Referring now to FIG. 6, a functional block diagram of a portion of an example implementation of the diabetes management device 104 is presented. The port 464 includes a connector 604 that includes a plurality of pins. Pins of a plug connect with the pins of the connector 604. As illustrated in the example of FIG. 6, the connector 604 may include a bus voltage (VBUS) pin, a ground pin (GRD), and two data pins (D+ and D-). While not shown in the example of FIG. 6, the connector 604 may also include an ID resistor pin that may be used, for example, to identify connection of one or more special types of cables or accessories. The connector 604 may include additional pins in various implementations. The diabetes management device 104 receives and transmits data via the data pins D+ and D-. The diabetes management device 104 receives a bus voltage (VBUS) via the bus voltage pin.

The diabetes management device 104 includes a multiplexer module 608, such as a USB multiplexer that is connected to the pins of the connector 604. The diabetes management device 104 may include a choke inductor 612 connected between the bus voltage pin and the multiplexer module 608.

The multiplexer module 608 includes multiplexer switching device. Multiplexer switching device means only that this switching device is included in the multiplexer. The multiplexer switching device is in particular a voltage and current protector switch 616. For example only, the protector switch 616 may include a field effect transistor (FET) or another suitable type of switching device. The bus voltage and/or current flow to the multiplexer module 608 may be monitored, and the protector switch 616 may be opened (i.e., open circuited) when the bus voltage is greater than a predetermined voltage and/or the current to the multiplexer module 608 is greater than a predetermined current. This may prevent one or more components of the diabetes management device 104 from being damaged in the event that over voltage and/or over current conditions occur.

When the bus voltage is less than the predetermined voltage and the current to the multiplexer module 608 is less than the predetermined current, the protector switch 616 may be closed. When the protector switch 616 is closed, the multiplexer module 608 outputs the bus voltage to a bus voltage node 620.

The diabetes management device 104 includes a processor module 624 that includes a processor 628. For example only, the processor module 624 may be an i.MX233 applications processor module. Code for executing functions of the handheld diabetes management device 104 is stored in memory 632. While the memory 632 is shown as being external to the processor module 624, the memory 632 may be wholly or partially integrated within the processor module 624.

The processor module 624 is directly connected to the bus voltage node 620. This connection provides an example of the second processor current path. In this manner, in the event that the battery 456 is lost or otherwise not connected to the diabetes management device 104, the diabetes management device 104 can be connected to another power source via the port 464 so a user can still at perform at least some of the functions of the diabetes management device 104.

The diabetes management device 104 includes a plurality of components for communicating between the diabetes management device 104 and one of a user of the diabetes management device 104 and another device (e.g., an insulin pump, a CGM, a computer, etc.). The components that communicate between the diabetes management device 104 and one of a user of the diabetes management device 104 and another device will be referred to as communication components. While examples of communication components are provided, the diabetes management device 104 may include other types of components that communicate information from the diabetes management device 104 to one of a user of the diabetes management device 104 and another device.

For example, the diabetes management device 104 includes the display 408 and one or more visual indicators, such as LED 444, that communicate information to a user of the diabetes management device 104. The diabetes management device 104 may also include a backlight 636 that backlights the display 408 for facilitating communication of information to a user. The diabetes management device 104 may also include a vibrator device 640 which vibrates to communicate information to a user.

The diabetes management device 104 also includes a communication module 644 that communicates with other devices. For example, the communication module 644 may communicate with a computer via the data pins of the connector 604. The communication module 644 may also communicate with other devices, such as wirelessly via one or more antennas (not shown).

When the battery 456 is not connected to the battery terminals 460, the processor module 624 may generally open a switching device provided as a first switch 648. The opening of the first switch 648 may be performed, for example, to reduce current drawn from the USB port 464. A reduction in current drawn from the USB port 464 may be required, for example, by a "low or dead battery" provision of a USB charging standard. If connection of a special cable is detected by the multiplexer module 608, for example via sensing a particular value of a resistor connected to the ID pin, the processor module 624 mat close the first switch 648 to enable operation without the battery 456. For example only, the first switch 648 may include a field effect transistor (FET) or another suitable type of switching device.

The first switch 648 is connected between the bus voltage node 620 and a second voltage node in the following named as a second node 652. A diode in the following named as a first diode 654 or another suitable device may be connected between the first switch 648 and the second node 652 to allow current flow from the bus voltage node 620 to the second node 652 and prevent current flow from the second node 652 back to the bus voltage node 620.

The communication components receive power via the second node 652. Closing the first switch 648 provides the bus voltage to the communication components. The processor module 624 determines whether (or not) the battery 456 is connected to the battery terminals 460 based on a battery status 688 generated by the monitoring module 684. The monitoring module 684 may sense whether a thermistor of the battery 456 is present and generate the battery status 688 based on whether the thermistor is present. For example, when the thermistor is not sensed, the monitoring module 684 may generate the battery status 688 to indicate that the battery 456 is not present, and vice versa. Other ways of determining whether the battery 457 is present, such as based on a voltage at a third voltage node, i.e. a third node 656, may be less reliable or unreliable due to the presence of multiple current leakage paths.

A second diode 660 or another suitable device may be connected between the second node 652 and a fourth node 664 to which the battery 456 is (or would be) connected to allow current flow from the fourth node 664 to the second node 652 and prevent current flow from the second node 652 back to the fourth node 664. The first diode 654 and the second diode 660 allow current to be supplied to the second node 652 from either the battery 456 or the bus voltage node 620. A third diode 668 or another suitable device may be connected between the fourth node 664 and the third node 656 to allow current flow from the fourth node 664 to the third node 656 and prevent current flow from the third node 656 to the fourth node 664.

A second switching device provided as a second switch 672 is connected between the bus voltage node 620 and the third node 656. For example only, the second switch 672 may include a FET or another suitable type of switching device. A fourth diode 676 or another suitable device may be connected between the second switch 672 and the third node 656 to allow current flow from the bus voltage node 620 (when the second switch 672 is closed) to the third node 656 and prevent current flow from the bus voltage node 620 to the third node 656. The third diode 668 and the fourth diode 676 may act as a logic OR function and allow either the battery 456 or the bus voltage node 620 to supply the voltage at the third node 656.

The processor module 624 may open the second switch 672 when the battery 456 is not connected to the battery terminals 460. The processor module 624 may open the second switch 672, for example, to disable a battery charger module 680 and to prevent excessive current draw from the bus voltage node 620. Opening the second switch 672 may prevent voltage and current from appearing at the battery terminals 460 which could be accessible by a user. When the battery 456 is connected to the battery terminals 460, the processor module 624 may close the second switch 672 and open the first switch 648. In this manner, the communication components and other components of the diabetes management device 104 receive power via the fourth node 664 when the battery 456 is connected to the battery terminals 460, and the current draw from the bus voltage node 620 is decreased.

The battery charger module 680 receives power from the bus voltage node 620 when the second switch 672 is closed. The battery charger module 680 selectively charges the battery 456 when the second switch 672 is closed. More specifically, the battery charger module 680 controls current flow to the battery 456.

The monitoring module 684 monitors one or more parameters. For example, the monitoring module 684 may monitor current flow to and/or from the battery 456, a voltage of the battery 456, a voltage at the fourth node 664, one or more temperatures of the battery 456, and/or other suitable parameters. The monitoring module 684 determines a state of charge (SOC) of the battery 456 based on one or more of the monitored parameters.

The battery charger module 680 controls current flow to the battery 456 based on a target current 692. Additionally or alternatively, the battery charger module 680 may control current flow to the battery 456 based on the SOC of the battery 456 and/or other input from the monitoring module 684. For example, the monitoring module 684 may selectively command the battery charger module 680 to not charge the battery 456 based on a temperature of the battery 456, and/or other suitable parameters.

A charge setting module 696 generates the target current 692. The charge setting module 696 may set the target current 692 based on signals from the multiplexer module 608 and/or the processor module 624. For example, the multiplexer module 608 may determine a predetermined value for the target current 692, and the processor module 624 may determine a present amount of power being consumed for operation of the diabetes management device 104. The charge setting module 696 may decrease the target current 692 from the predetermined value based on the present amount of power being consumed for operation of the diabetes management device 104. The processor module 624 and the battery charger module 680 may determine the predetermined value for the target current 692, for example, as a function of temperature of the battery 456, voltage of the battery 456, the SOC of the battery 456, and/or one or more other suitable parameters.

As stated above, the processor module 624 receives power via the bus voltage node 620 and/or the third node 656, and the communication components receive power via the second node 652 and/or the fourth node 664. In this manner, the processor module 624 is operational and the communication components can be operated, even at times when the battery 456 is not connected to the battery terminals 460.

The processor module 624 may control operation of the communication module 644 via a first driver 698. The processor module 624 may control operation of the backlight 636 via a second driver 700. The processor module 624 may control operation of the LED 444 via a third driver 704. The processor module 624 may control operation of the vibrator device 640 via a fourth driver 708. The processor module 624 may control operation of the display 408 via a fifth driver 712. The processor module 624 may control operation of the speaker 448 via a sixth driver 716.

A measurement engine 720 may receive power via the third node 656. Thus, the measurement engine 720 may only be operable when the battery 456 is connected to the battery terminals 460. The measurement engine 720 may also receive power via the bus voltage node 620 when the second switch 672 is closed by the processor module 624.

The processor module 624 may control operation of the measurement engine 720 via a seventh driver 724. The measurement engine 720 may determine a bG level of a blood sample when a bG test strip, such as the bG test strip 416, is inserted into the bG test strip port 420. The monitoring module 684 may also receive power via the third node 656, and the processor module 624 may control operation of the monitoring module 684 via an eighth driver 728.

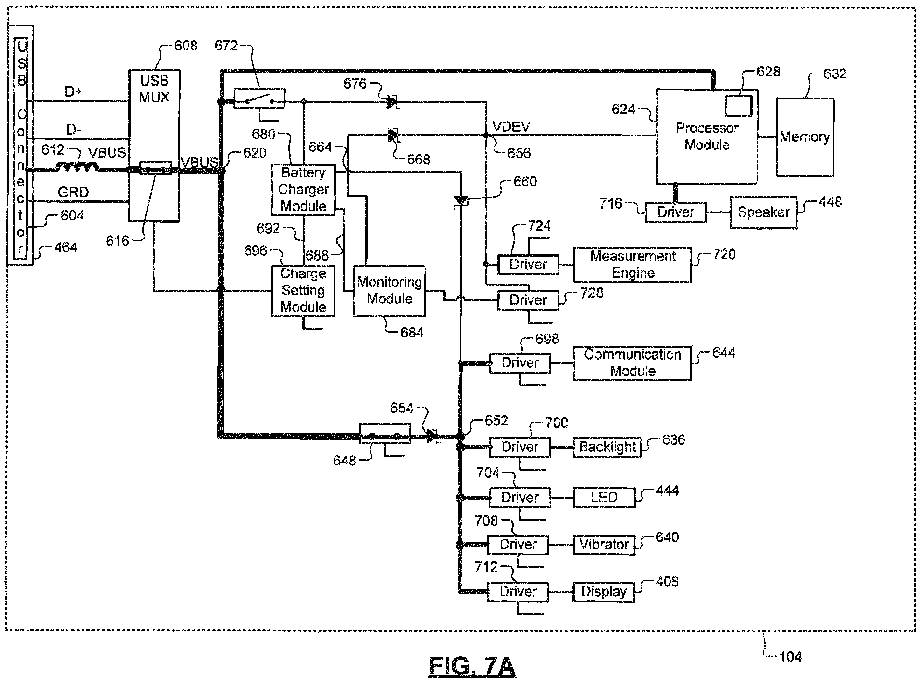

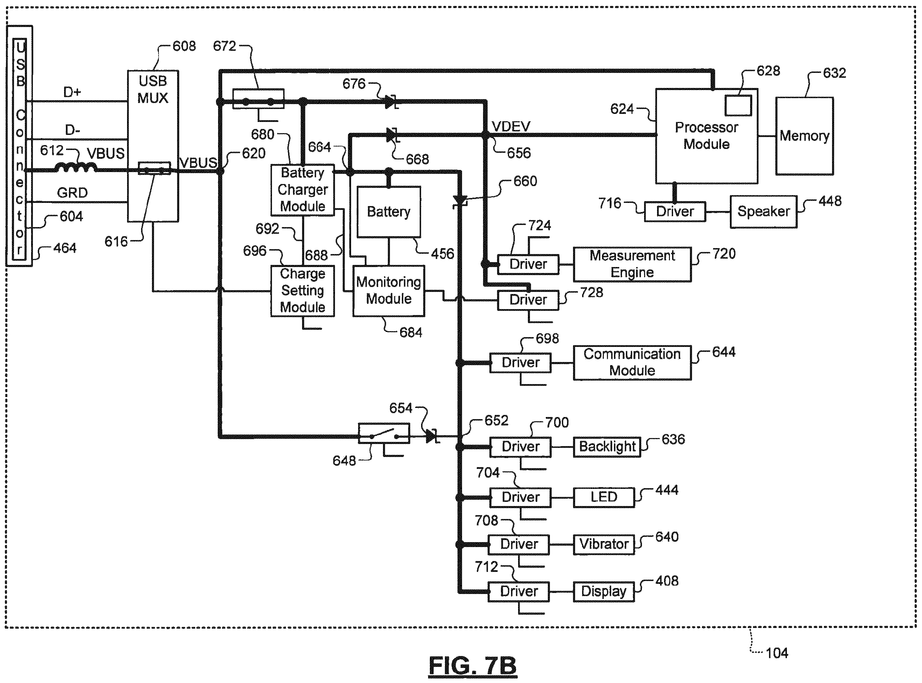

FIG. 7A is a functional block diagram of the portion of the example implementation of the diabetes management device 104. The bold lines in FIG. 7A illustrate an example current path when the battery 456 is not connected to the battery terminals 460. The battery 456 is not shown in the example of FIG. 7A to emphasize this. The bold lines in FIG. 7B illustrate a further example current path when the battery 456 is connected to the battery terminals 460.

As illustrated by FIGs. 7A and 7B, the communication devices may be operated at times when the battery 456 is not connected to the battery terminals 460 and at times when the battery 456 is connected to the battery terminals 460. This may enable a user to perform various functions of the diabetes management device 104 even at times when the battery 456 is not connected to the battery terminals 460. For example, the user may review previously recorded diabetes management data (e.g., bG measurements, activity, insulin administration, food intake, etc.), communicate diabetes management data to another device (e.g., a personal computer), and perform other functions of the diabetes management device 104.

The measurement engine 720 and the monitoring module 684, however, may only be operated at times when the battery 456 is connected to the battery terminals 460 or when the processor module 624 closes the second switch 672 to power the measurement engine 720 and the monitoring module 684 at times when a special cable is connected to the USB port 464. Operation of the measurement engine 720 may be limited to times when the battery 456 is connected to the battery terminals 460, for example, to ensure that bG measurements are as accurate as possible. bG measurements taken while the battery 456 is not connected to the battery terminals 460 and power is received via the port 464 may cause electrical safety concerns. Operation of the monitoring module 684 may be limited to times when the battery 456 is connected to the battery terminals 460, for example, to limit unnecessary power consumption when the battery 456 is not present.

In a feature, a handheld diabetes management device that is at least partially functional while a battery of the handheld diabetes management device is disconnected from the handheld diabetes management device and power is supplied to the handheld diabetes management device via a universal serial bus (USB) connection is described. The handheld diabetes management device includes: a processor module including memory having instructions for executing functions of the handheld diabetes management device and a processor that selectively executes the instructions; and battery terminals that electrically connect the processor module with a, in particular re-chargeable, battery when the battery is inserted within a battery compartment of the handheld diabetes management device. This connection provides an example of the first processor current path The handheld diabetes management device further includes: a receptacle that is configured in accordance with a universal serial bus (USB) standard and that receives a plug that is configured in accordance with the USB standard; at least one component for communicating information from the handheld diabetes management device to one of a user and another device; and a multiplexer module that receives power via the receptacle when the plug is connected with the receptacle and that selectively outputs power to a bus voltage node based on the power received via the receptacle. The processor module is directly connected to the bus voltage node and receives power from the bus voltage node when the re-chargeable battery is not connected to the battery terminals. The at least one component receives power via the bus voltage node when the rechargeable battery is not connected to the battery terminals. The processor module controls application of power to the at least one component and operation of the at least one component when the re-chargeable battery is not connected to the battery terminals.

In further features, the multiplexer module includes a multiplexer switching device that is connected between the receptacle and the bus voltage node. The multiplexer switching device is open circuited when the power output to the bus voltage node is greater than a predetermined value.

In still further features, the handheld diabetes management device further includes a switching device that is connected between the bus voltage node and a second voltage node. The at least one component receives power from the second voltage node.

In other features, the processor module closes the switching device in response to a determination that the re-chargeable battery is not connected to the battery terminals.

In still other features, the handheld diabetes management device further includes a diode that is connected between the switching device and the second voltage node, that allows current flow from the switching device to the second voltage node, and that blocks current flow from the second voltage node to the switching device.

In further features, the handheld diabetes management device further includes a second switching device that is connected between the bus voltage node and a third voltage node. One of the battery terminals is connected to the third voltage node, and the processor module is also directly connected to the third voltage node.

In still further features, the handheld diabetes management device further includes: a blood glucose (bG) test strip port; and a bG measurement engine that is connected to the third voltage node and that measures a bG level of a bG test strip when the bG test strip is inserted into the bG test strip port. The at least one component includes a communication module that communicates data from the handheld diabetes management to another device.

In other features, the re-chargeable battery is connected to the battery terminals, and the processor module selectively opens and closes the switching device and the second switching device. When the re-chargeable battery is not connected to the battery terminals, the processor module selectively opens and closes the switching device and the second switching device.

In still other features, when the re-chargeable battery is connected to the battery terminals, the processor module opens the switching device and closes the second switching device. When the re-chargeable battery is not connected to the battery terminals, the processor module closes the switching device and opens the second switching device.

In further features, the handheld diabetes management device further includes a battery charger module that receives power output from the second switching device and that, based on power consumption of the at least one component, controls current flow to the rechargeable battery for re-charging the re-chargeable battery.

In still further features, the handheld diabetes management device further includes: a first processor current path from the re-chargeable battery to the processor module; and a second processor current path from the receptacle to the processor module via the multiplexer module and the bus voltage node. When the re-chargeable battery is not connected to the battery terminals, the processor module receives power via the second processor current path.

The handheld diabetes management device further includes: a first current path from the battery to the at least one component; and a second current path from the bus voltage node to the at least one component.

In still other features, in response to a determination that the re-chargeable battery is connected to the battery terminals, the processor module enables current flow through the first current path and disables current flow through the second current path; and in response to a determination that the re-chargeable battery is not connected to the battery terminals, the processor module disables current flow through the first current path and enables current flow through the second current path.

In further features, in response to a determination that the re-chargeable battery is not connected to the battery terminals, the processor module disables current flow through the first current path and enables current flow through the second current path.

In still further features, the at least one component includes a touchscreen display, a backlight of the touchscreen display, a visual indicator, and a vibrator device.

In another feature, a handheld diabetes management device that is at least partially functional while a battery of the handheld diabetes management device is disconnected from the handheld diabetes management device and power is supplied to the handheld diabetes management device via a universal serial bus (USB) connection is described. The handheld diabetes management device includes: a processor module including memory having instructions for executing functions of the handheld diabetes management device and a processor that selectively executes the instructions; battery terminals that electrically connect the processor module with a re-chargeable battery when the re-chargeable battery is inserted within a battery compartment of the handheld diabetes management device; and a receptacle that is configured in accordance with a universal serial bus (USB) standard and that receives a plug that is configured in accordance with the USB standard. The handheld diabetes management device further includes: at least one component for communicating information from the handheld diabetes management device to one of a user and another device; a multiplexer module that receives power via the receptacle when the plug is connected with the receptacle and that relays power from the receptacle to a bus voltage node, wherein the processor module is directly connected to the bus voltage node; a first current path from the battery to the at least one component; and a second current path from the bus voltage node to the at least one component. In response to a determination that the re-chargeable battery is connected to the battery terminals, the processor module enables current flow through the first current path and disables current flow through the second current path. In response to a determination that the re-chargeable battery is not connected to the battery terminals, the processor module disables current flow through the first current path and enables current flow through the second current path.

In further features, the handheld diabetes management device further includes a battery charger module that receives power from the bus voltage node and that, based on power consumption of the at least one component, controls current flow to the re-chargeable battery for re-charging the re-chargeable battery.

In still further features, the handheld diabetes management device further includes: a blood glucose (bG) test strip port; and a bG measurement engine that measures a bG level of a bG test strip when the bG test strip is inserted into the bG test strip port.

In another feature, a handheld diabetes management device that is at least partially functional while a battery of the handheld diabetes management device is disconnected from the handheld diabetes management device and power is supplied to the handheld diabetes management device via a universal serial bus (USB) connection is described. The handheld diabetes management device includes: a processor module including memory having instructions for executing functions of the handheld diabetes management device and a processor that selectively executes the instructions; battery terminals that electrically connect the processor module with a re-chargeable battery when the re-chargeable battery is inserted within a battery compartment of the handheld diabetes management device; and a receptacle that is configured in accordance with a universal serial bus (USB) standard and that receives a plug that is configured in accordance with the USB standard. The handheld diabetes management device further includes: at least one component for communicating information from the handheld diabetes management device to one of a user and another device; a multiplexer module that receives power via the receptacle when the plug is connected with the receptacle and that relays power from the receptacle to a bus voltage node, wherein the processor module is directly connected to the bus voltage node; a switching device, i.e. a first switching device, that is connected between the bus voltage node and a second voltage node, wherein the at least one component receives power from the second voltage node; and a second switching device that is connected between the bus voltage node and a third voltage node. One of the battery terminals is connected to the third voltage node, and the processor module is also directly connected to the third voltage node.

In further features, the handheld diabetes management device further includes: a blood glucose (bG) test strip port; and a bG measurement engine that is connected to the third voltage node and that measures a bG level of a bG test strip when the bG test strip is inserted into the bG test strip port. The at least one component includes a communication module that communicates data from the handheld diabetes management to another device. In response to a determination that the re-chargeable battery is connected to the battery terminals, the processor module closes the second switching device and opens the first switching device. In response to a determination that the re-chargeable battery is not connected to the battery terminals, the processor module opens the second switching device and closes the first switching device.

As used herein, the term "module" can refer to, be part of, or include an Application Specific Integrated Circuit (ASIC); an electronic circuit; a combinational logic circuit; a field programmable gate array (FPGA); a processor (shared, dedicated, or group) that executes code; other suitable components that provide the described functionality; or a combination of some or all of the above, such as in a system-on-chip. The term "module" can include memory (shared, dedicated, or group) that stores code executed by the processor.

The term "code," as used above, can include software, firmware, and/or microcode, and can refer to programs, routines, functions, classes, and/or objects. The term "shared," as used above, means that some or all code from multiple modules can be executed using a single (shared) processor. In addition, some or all code from multiple modules can be stored by a single (shared) memory. The term "group," as used above, means that some or all code from a single module can be executed using a group of processors. In addition, some or all code from a single module can be stored using a group of memories.

The apparatuses and methods described herein can be implemented by one or more computer programs executed by one or more processors. The computer programs include processor-executable instructions that are stored on a non-transitory, tangible, computer readable medium. The computer programs can also include stored data. Examples of the non-transitory, tangible, computer readable medium include, but are not limited to, nonvolatile memory, magnetic storage, and optical storage.

The description is merely illustrative in nature and is in no way intended to limit the disclosure, its application, or uses. For purposes of clarity, the same reference numbers may be used in the drawings to identify similar elements. As used herein, the phrase at least one of A, B, and C should be construed to mean a logical (A or B or C), using a non-exclusive logical or. It should be understood that steps within a method can be executed in different order without altering the principles of the present disclosure.

The broad teachings of the disclosure can be implemented in a variety of forms. Therefore, while this disclosure includes particular examples, the true scope of the disclosure should not be so limited since other modifications will become apparent to the skilled practitioner upon a study of the drawings, the specification, and the following claims

## Claims

1. A handheld diabetes management device (104) comprising:
a processor module (624) including memory (632) having instructions for executing functions of the handheld diabetes management device (104) and a processor (628) that selectively executes the instructions;
battery terminals (460) that electrically connect the processor module (624) with a battery (456) when the battery is inserted within a battery compartment of the handheld diabetes management device (104);
a receptacle that in particular is configured in accordance with a universal serial bus (USB) standard and that receives a plug that in particular is configured in accordance with the USB standard;
at least one component (408,444,636,640) configured to communicating information from the handheld diabetes management device (104) to one of a user and another device;
a monitoring module (684) configured to generate a battery status (688); and
a multiplexer module (608) configured to receive power via the receptacle when the plug is connected with the receptacle and to relay power from the receptacle to a bus voltage node (620),
in particular the multiplex module (608) is configured to selectively output power to the bus voltage node (620) based on the power received via the receptacle,
wherein the processor module (624) is directly connected to the bus voltage node (620), wherein the handheld diabetes management device (104) comprises a first current path from the battery (456) to the at least one component (408,444,636,640); and a second current path from the bus voltage node (620) to the at least one component (408,444,636,640), and
wherein the processor module (624) is configured to
enable current flow through the first current path and disable current flow through the second current path in response to a determination, based on the battery status (688) generated by the monitoring module (684), that the battery (456) is connected to the battery terminals (460); and
to disable current flow through the first current path and enable current flow through the second current path in response to a determination, based on the battery status (688) generated by the monitoring module (684), that the battery (456) is not connected to the battery terminals (460).

2. The diabetes management device of claim 1,
wherein the processor module (624) is configured to control application of power to the at least one component (408,444,636,640) and operation of the at least one component (408,444,636,640) when the battery (456) is not connected to the battery terminals (460),
in particular wherein the at least one component (408,444,636,640) is configured to receive power via the bus voltage node (620) when the battery (456) is not connected to the battery terminals (460).

3. The diabetes management device of claim 1 or 2 wherein the multiplexer module (608) includes a multiplexer switching device (616) that is connected between the receptacle and the bus voltage node (620), and
wherein the multiplexer switching device (616) is configured to be open circuited when the power output to the bus voltage node (620) is greater than a predetermined value.

4. The handheld diabetes management device (104) according to any of the preceding claims further comprising a switching device (648) that is connected between the bus voltage node (620) and a second voltage node (652),
wherein the at least one component (408,444,636,640) receives power from the second voltage node (652).

5. The handheld diabetes management device (104) of claim 4 wherein the processor module (624) is configured to close the switching device (648) in response to a determination that the battery (456) is not connected to the battery terminals (460).

6. The handheld diabetes management device (104) of claim 4 or 5 further comprising a diode (654) that is connected between the switching device (648) and the second voltage node (652), so that currentflow from the switching device (648) to the second voltage node (652) is allowed, and current flow from the second voltage node (652) to the switching device (648) is blocked.

7. The handheld diabetes management device (104) according to any of the preceding claims, further comprising a second switching device (672) that is connected between the bus voltage node (620) and a third voltage node (656),
wherein one of the battery terminals (460) is connected to the third voltage node (656), and
wherein the processor module (624) is also, in particular directly, connected to the third voltage node (656).

8. The handheld diabetes management device (104) according to any of the preceding claims, further comprising:
a blood glucose (bG) test strip port (420); and
a bG measurement engine (720) that is connected to the third voltage node (656) and
that measures a bG level of a bG test strip (416) when the bG test strip (416) is inserted into the bG test strip port (420),
wherein the at least one component (408,444,636,640) includes a communication module (644) that is configured to communicate data from the handheld diabetes management device (104) to another device.

9. The handheld diabetes management device (104) of claim 7 or, 7 and 8, wherein:
when the battery (456) is connected to the battery terminals (460), the processor module (624) is configured to selectively open and close the switching device (648) and the second switching device (672); and
when the battery (456) is not connected to the battery terminals (460), the processor module is configured to selectively open and close the switching device (648) and the second switching device (672),
wherein the processor module (624) is configured to open the switching device (648) and closes the second switching device (672) when the battery (456) is connected to the battery terminals (460), and
close the switching device (648) and open the second switching device (672) when the battery (456) is not connected to the battery terminals (460).

10. The handheld diabetes management device (104) according to any of the preceding claims, wherein the battery (456) is a re-chargeable battery and wherein the handheld diabetes management device (104) further comprising a battery charger module (680) that is configured to receive power output from the second switching device (672) and, control current flow to the re-chargeable battery for re-charging the re-chargeable battery, based on power consumption of the at least one component (408,444,636,640).

11. The handheld diabetes management device (104) according to any of the preceding claims further comprising:
a first processor current path from the battery (456) to the processor module (624); and
a second processor current path from the receptacle to the processor module (624) via the multiplexer module (608) and the bus voltage node (620),
wherein, the processor module (624) is configured to receive power via the second current path when the battery (456) is not connected to the battery terminals (460).

12. The handheld diabetes management device (104) according to any of the preceeding claims wherein the at least one component (408,444,636,640) includes a touchscreen display (408), a backlight (636) of the touchscreen display, a visual indicator (444), and a vibrator device (640).

## Patentansprüche

1. Tragbare Diabetesbehandlungsvorrichtung (104), umfassend:
ein Prozessormodul (624), das einen Speicher (632) mit Anweisungen zum Ausführen von Funktionen der tragbaren Diabetesbehandlungsvorrichtung (104) und einen Prozessor (628), der die Anweisungen selektiv ausführt, einschließt;
Batterieklemmen (460), die das Prozessormodul (624) elektrisch an eine Batterie (456) anschließen, wenn die Batterie in ein Batteriefach der tragbaren Diabetesbehandlungsvorrichtung (104) eingelegt wird;
eine Steckbuchse, die insbesondere nach einem Universal-Serial-Bus-(USB)-Standard konfiguriert ist und die einen Stecker aufnimmt, der insbesondere nach dem USB-Standard konfiguriert ist;
mindestens eine Komponente (408, 444, 636, 640), die dafür ausgelegt ist, Informationen von der tragbaren Diabetesbehandlungsvorrichtung (104) an einen Nutzer oder eine andere Vorrichtung zu übertragen;
ein Überwachungsmodul (684), das dafür ausgelegt ist, einen Batteriestatus (688) zu erzeugen; und
ein Multiplexer-Modul (608), das dafür ausgelegt ist, über die Steckbuchse Energie aufzunehmen, wenn der Stecker an die Steckbuchse angeschlossen wird, und Energie aus der Steckbuchse an einen Bus-Spannungsknoten (620) weiterzuleiten,
insbesondere ist das Multiplexer-Modul (608) dafür ausgelegt, basierend auf der über die Steckbuchse aufgenommene Energie selektiv Energie an den Bus-Spannungsknoten (620) abzugeben,
wobei das Prozessormodul (624) direkt an den Bus-Spannungsknoten (620) angeschlossen ist, wobei die tragbare Diabetesbehandlungsvorrichtung (104) eine erste Strombahn von der Batterie (456) zu der mindestens einen Komponente (408, 444, 636, 640) und eine zweite Strombahn von dem Bus-Spannungsknoten (620) zu der mindestens einen Komponente (408, 444, 636, 640) umfasst, und
wobei das Prozessormodul (624) dafür ausgelegt ist,
als Reaktion auf eine Bestimmung, basierend auf dem von dem Überwachungsmodul (684) erzeugten Batteriestatus (688), dass die Batterie (456) an die Batterieklemmen (460) angeschlossen ist, Stromfluss durch die erste Strombahn freizugeben und Stromfluss durch die zweite Strombahn abzuschalten; und
als Reaktion auf eine Bestimmung, basierend auf dem von dem Überwachungsmodul (684) erzeugten Batteriestatus (688), dass die Batterie (456) nicht an die Batterieklemmen (460) angeschlossen ist, Stromfluss durch die erste Strombahn abzuschalten und Stromfluss durch die zweite Strombahn freizugeben.

2. Diabetesbehandlungsvorrichtung nach Anspruch 1,
wobei das Prozessormodul (624) dafür ausgelegt ist, das Anlegen von Energie an die mindestens eine Komponente (408, 444, 636, 640) und den Betrieb der mindestens einen Komponente (408, 444, 636, 640) zu steuern, wenn die Batterie (456) nicht an die Batterieklemmen (460) angeschlossen ist,
insbesondere wobei die mindestens eine Komponente (408, 444, 636, 640) dafür ausgelegt ist, über den Bus-Spannungsknoten (620) Energie aufzunehmen, wenn die Batterie (456) nicht an die Batterieklemmen (460) angeschlossen ist.

3. Diabetesbehandlungsvorrichtung nach Anspruch 1 oder 2, wobei das Multiplexer-Modul (608) eine Multiplexer-Schaltvorrichtung (616) einschließt, die zwischen der Steckbuchse und dem Bus-Spannungsknoten (620) angeschlossen ist, und
wobei die Multiplexer-Schaltvorrichtung (616) dafür ausgelegt ist, auf Leerlauf geschaltet werden zu können, wenn die an den Bus-Spannungsknoten (620) abgegebene Energie größer ist als ein vorbestimmter Wert.

4. Tragbare Diabetesbehandlungsvorrichtung (104) nach einem der vorstehenden Ansprüche, ferner umfassend eine Schaltvorrichtung (648), die zwischen dem Bus-Spannungsknoten (620) und einem zweiten Spannungsknoten (652) angeschlossen ist,
wobei die mindestens eine Komponente (408, 444, 636, 640) Energie von dem zweiten Spannungsknoten (652) aufnimmt.

5. Tragbare Diabetesbehandlungsvorrichtung (104) nach Anspruch 4, wobei das Prozessormodul (624) dafür ausgelegt ist, die Schaltvorrichtung (648) als Reaktion auf eine Bestimmung, dass die Batterie (456) nicht an die Batterieklemmen (460) angeschlossen ist, zu schließen.

6. Tragbare Diabetesbehandlungsvorrichtung (104) nach Anspruch 4 oder 5, ferner umfassend eine Diode (654), die zwischen der Schaltvorrichtung (648) und dem zweiten Spannungsknoten (652) angeschlossen ist, so dass Stromfluss von der Schaltvorrichtung (648) zu dem zweiten Spannungsknoten (652) möglich ist und Stromfluss vom zweiten Spannungsknoten (652) zur Schaltvorrichtung (648) blockiert ist.

7. Tragbare Diabetesbehandlungsvorrichtung (104) nach einem der vorstehenden Ansprüche, ferner umfassend eine zweite Schaltvorrichtung (672), die zwischen dem Bus-Spannungsknoten (620) und einem dritten Spannungsknoten (656) angeschlossen ist,
wobei eine der Batterieklemmen (460) an den dritten Spannungsknoten (656) angeschlossen ist und
wobei auch das Prozessormodul (624) insbesondere direkt an den dritten Spannungsknoten (656) angeschlossen ist.

8. Tragbare Diabetesbehandlungsvorrichtung (104) nach einem der vorstehenden Ansprüche, ferner umfassend:
einen Blutglucose-(bG)-Teststreifenschlitz (420) und
eine bG-Messfunktionseinheit (720), die an den dritten Spannungsknoten (656) angeschlossen ist und
die einen bG-Spiegel eines bG-Teststreifens (416) misst, wenn der bG-Teststreifen (416) in den bG-Teststreifenschlitz (420) eingeführt wird,
wobei die mindestens eine Komponente (408, 444, 636, 640) ein Kommunikationsmodul (644) einschließt, das dafür ausgelegt ist, Daten von der tragbaren Diabetesbehandlungsvorrichtung (104) an eine andere Vorrichtung zu übertragen.

9. Tragbare Diabetesbehandlungsvorrichtung (104) nach Anspruch 7 oder 7 und 8, wobei:
wenn die Batterie (456) an die Batterieklemmen (460) angeschlossen ist, das Prozessormodul (624) dafür ausgelegt ist, die Schaltvorrichtung (648) und die zweite Schaltvorrichtung (672) selektiv zu öffnen und zu schließen; und
wenn die Batterie (456) nicht an die Batterieklemmen (460) angeschlossen ist, das Prozessormodul dafür ausgelegt ist, die Schaltvorrichtung (648) und die zweite Schaltvorrichtung (672) selektiv zu öffnen und zu schließen,
wobei das Prozessormodul (624) dafür ausgelegt ist, die Schaltvorrichtung (648) zu öffnen und die zweite Schaltvorrichtung (672) zu schließen, wenn die Batterie (456) an die Batterieklemmen (460) angeschlossen ist, und
die Schaltvorrichtung (648) zu schließen und die zweite Schaltvorrichtung (672) zu öffnen, wenn die Batterie (456) nicht an die Batterieklemmen (460) angeschlossen ist.

10. Tragbare Diabetesbehandlungsvorrichtung (104) nach einem der vorstehenden Ansprüche, wobei die Batterie (456) eine wiederaufladbare Batterie ist und wobei die tragbare Diabetesbehandlungsvorrichtung (104) ferner ein Batterielademodul (680) umfasst, das dafür ausgelegt ist, aus der zweiten Schaltvorrichtung (672) abgegebene Energie aufzunehmen und Stromfluss zu der wiederaufladbaren Batterie zum Wiederaufladen der wiederaufladbaren Batterie basierend auf dem Energieverbrauch der mindestens einen Komponente (408, 444, 636, 640) zu steuern.

11. Tragbare Diabetesbehandlungsvorrichtung (104) nach einem der vorstehenden Ansprüche, ferner umfassend:
eine erste Prozessorstrombahn von der Batterie (456) zum Prozessormodul (624) und
eine zweite Prozessorstrombahn von der Steckbuchse zum Prozessormodul (624) über das Multiplexer-Modul (608) und den Bus-Spannungsknoten (620),
wobei das Prozessormodul (624) dafür ausgelegt ist, Energie über die zweite Strombahn aufzunehmen, wenn die Batterie (456) nicht an die Batterieklemmen (460) angeschlossen ist.

12. Tragbare Diabetesbehandlungsvorrichtung (104) nach einem der vorstehenden Ansprüche, wobei die mindestens eine Komponente (408, 444, 636, 640) eine Touchscreen-Anzeige (408), eine Hintergrundbeleuchtung (636) der Touchscreen-Anzeige, einen visuellen Indikator (444) und eine Vibrationsvorrichtung (640) einschließt.

## Revendications

1. Dispositif de gestion du diabète manuel (104) comprenant :
un module de processeur (624) incluant une mémoire (632) ayant des instructions pour exécuter des fonctions du dispositif de gestion du diabète manuel (104) et un processeur (628) qui exécute sélectivement les instructions ;
des bornes de batterie (460) qui connectent électriquement le module de processeur (624) à une batterie (456) lorsque la batterie est insérée dans un compartiment de batterie du dispositif de gestion du diabète manuel (104) ;
une prise qui en particulier est configurée conformément à une norme de bus universel en série (USB) et qui reçoit une fiche qui en particulier est configurée conformément à la norme USB ;
au moins un composant (408, 444, 636, 640) configuré pour communiquer des informations du dispositif de gestion du diabète manuel (104) à l'un parmi un utilisateur et un autre dispositif ;
un module de surveillance (684) configuré pour générer un état de batterie (688) ; et
un module de multiplexeur (608) configuré pour recevoir de l'énergie par le biais de la prise lorsque la fiche est connectée à la prise et pour transmettre l'énergie de la prise à un nœud de tension de bus (620),
en particulier le module de multiplexeur (608) est configuré pour délivrer sélectivement de l'énergie au nœud de tension de bus (620) sur la base de l'énergie reçue par le biais de la prise,
dans lequel le module de processeur (624) est directement connecté au nœud de tension de bus (620), dans lequel le dispositif de gestion du diabète manuel (104) comprend un premier trajet de courant de la batterie (456) jusqu'à au moins un composant (408, 444, 636, 640) ; et un second trajet de courant du nœud de tension de bus (620) jusqu'à l'au moins un composant (408, 444, 636, 640), et
dans lequel le module de processeur (624) est configuré pour
activer la circulation du courant à travers le premier trajet de courant et désactiver la circulation du courant à travers le second trajet de courant en réponse à une détermination, sur la base de l'état de batterie (688) généré par le module de surveillance (684), que la batterie (456) est connectée aux bornes de batterie (460) ; et
désactiver la circulation du courant à travers le premier trajet de courant et activer la circulation du courant à travers le second trajet de courant en réponse à une détermination, sur la base de l'état de batterie (688) généré par le module de surveillance (684), que la batterie (456) n'est pas connectée aux bornes de batterie (460).

2. Dispositif de gestion du diabète selon la revendication 1,
dans lequel le module de processeur (624) est configuré pour commander l'application d'énergie à l'au moins un composant (408, 444, 636, 640) et le fonctionnement de l'au moins un composant (408, 444, 636, 640) lorsque la batterie (456) n'est pas connectée aux bornes de batterie (460),
en particulier dans lequel l'au moins un composant (408, 444, 636, 640) est configuré pour recevoir de l'énergie par le biais du nœud de tension de bus (620) lorsque la batterie (456) n'est pas connectée aux bornes de batterie (460).

3. Dispositif de gestion du diabète selon la revendication 1 ou 2 dans lequel le module de multiplexeur (608) inclut un dispositif de commutation de multiplexeur (616) qui est connecté entre la prise et le nœud de tension de bus (620), et
dans lequel le dispositif de commutation de multiplexeur (616) est configuré pour être en circuit ouvert lorsque l'énergie délivrée au nœud de tension de bus (620) est supérieure à une valeur prédéterminée.

4. Dispositif de gestion du diabète manuel (104) selon l'une quelconque des revendications précédentes comprenant en outre un dispositif de commutation (648) qui est connecté entre le nœud de tension de bus (620) et un deuxième nœud de tension (652),
dans lequel l'au moins un composant (408, 444, 636, 640) reçoit de l'énergie du deuxième nœud de tension (652).

5. Dispositif de gestion du diabète manuel (104) selon la revendication 4 dans lequel le module de processeur (624) est configuré pour fermer le dispositif de commutation (648) en réponse à une détermination que la batterie (456) n'est pas connectée aux bornes de batterie (460).

6. Dispositif de gestion du diabète manuel (104) selon la revendication 4 ou 5 comprenant en outre une diode (654) qui est connectée entre le dispositif de commutation (648) et le deuxième nœud de tension (652), de sorte que la circulation du courant du dispositif de commutation (648) jusqu'au deuxième nœud de tension (652) est permise, et que la circulation du courant du deuxième nœud de tension (652) jusqu'au dispositif de commutation (648) est bloquée.

7. Dispositif de gestion du diabète manuel (104) selon l'une quelconque des revendications précédentes, comprenant en outre un second dispositif de commutation (672) qui est connecté entre le nœud de tension de bus (620) et un troisième nœud de tension (656),
dans lequel l'une des bornes de batterie (460) est connectée au troisième nœud de tension (656), et
dans lequel le module de processeur (624) est également, en particulier directement, connecté au troisième nœud de tension (656).

8. Dispositif de gestion du diabète manuel (104) selon l'une quelconque des revendications précédentes, comprenant en outre :
un orifice pour bandelette de test de glycémie sanguine (bG) (420) ; et
un moteur de mesure de bG (720) qui est connecté au troisième nœud de tension (656) et
qui mesure un taux de bG d'une bandelette de test de bG (416) lorsque la bandelette de test de bG (416) est insérée dans l'orifice pour bandelette de test de bG (420),
dans lequel l'au moins un composant (408, 444, 636, 640) inclut un module de communication (644) qui est configuré pour communiquer des données du dispositif de gestion du diabète manuel (104) à un autre dispositif.

9. Dispositif de gestion du diabète manuel (104) selon la revendication 7, ou 7 et 8, dans lequel :
lorsque la batterie (456) est connectée aux bornes de batterie (460), le module de processeur (624) est configuré pour ouvrir et fermer sélectivement le dispositif de commutation (648) et le second dispositif de commutation (672) ; et
lorsque la batterie (456) n'est pas connectée aux bornes de batterie (460), le module de processeur est configuré pour ouvrir et fermer sélectivement le dispositif de commutation (648) et le second dispositif de commutation (672),
dans lequel le module de processeur (624) est configuré pour ouvrir le dispositif de commutation (648) et fermer le second dispositif de commutation (672) lorsque la batterie (456) est connectée aux bornes de batterie (460), et
fermer le dispositif de commutation (648) et ouvrir le second dispositif de commutation (672) lorsque la batterie (456) n'est pas connectée aux bornes de batterie (460).

10. Dispositif de gestion du diabète manuel (104) selon l'une quelconque des revendications précédentes, dans lequel la batterie (456) est une batterie rechargeable et dans lequel le dispositif de gestion du diabète manuel (104) comprend en outre un module de chargeur de batterie (680) qui est configuré pour recevoir une énergie délivrée par le second dispositif de commutation (672), et commander la circulation du courant jusqu'à la batterie rechargeable pour recharger la batterie rechargeable, sur la base de la consommation d'énergie de l'au moins un composant (408, 444, 636, 640).

11. Dispositif de gestion du diabète manuel (104) selon l'une quelconque des revendications précédentes comprenant en outre :
un premier trajet de courant de processeur de la batterie (456) jusqu'au module de processeur (624) ; et
un second trajet de courant de processeur de la prise jusqu'au module de processeur (624) par le biais du module de multiplexeur (608) et du nœud de tension de bus (620),
dans lequel, le module de processeur (624) est configuré pour recevoir de l'énergie par le biais du second trajet de courant lorsque la batterie (456) n'est pas connectée aux bornes de batterie (460).

12. Dispositif de gestion du diabète manuel (104) selon l'une quelconque des revendications précédentes dans lequel l'au moins un composant (408, 444, 636, 640) inclut un affichage à écran tactile (408), un rétroéclairage (636) de l'affichage à écran tactile, un indicateur visuel (444) et un dispositif vibrant (640).
